# EUROPÄISCHE PATENTSCHRIFT

(11) **EP 0 772 584 B1**
(45) Veröffentlichungstag und Bekanntmachung des Hinweises auf die Patenterteilung: **17.06.1998**
(21) Anmeldenummer: 95931978.1
(22) Anmeldetag: 02.09.1995
(51) Int. Cl.: C07C 211/27, C07C 209/08

(54) **VERFAHREN ZUR HERSTELLUNG VON SELEGILIN**
PROCESS FOR PREPARING SELEGILIN
PROCEDE DE FABRICATION DE SELEGILINE

(30) Priorität: 13.09.1994 DE 4432610
(43) Veröffentlichungstag der Anmeldung: 14.05.1997
(73) Patentinhaber: KNOLL AKTIENGESELLSCHAFT, D-67061 Ludwigshafen (DE)
(72) Erfinder: OTT-DEMBROWSKI, Silvia, D-32459 Petershagen (DE); CYRUS, Richard, D-67067 Ludwigshafen (DE); SCHMIDT, Jörg, D-32427 Minden (DE); WAIBLINGER, Hans, D-32547 Bad Oeynhausen (DE)
(74) Vertreter: Karau, Wolfgang, Dr.
(86) Internationale Anmeldenummer: EP9503460
(87) Internationale Veröffentlichungsnummer: WO9608461

(56) Entgegenhaltungen:
- EP-A- 0 099 302
- EP-A- 0 344 675
- CHEMICAL ABSTRACTS, vol. 105, no. 13, 29. September 1986, Columbus, Ohio, US; abstract no. 114597s, Z. ECSERY ET. AL. 'Pharmaceutical Propargylamines' Seite 632 ;Spalte 2 ; & HU,A,35 630

## Beschreibung

Die vorliegende Erfindung betrifft ein neues Verfahren zur Herstellung von Selegilin.

Selegilin ist das (R)-(-)-Isomere des Deprenyls (= N,∼-DimethylN-2-propinylphenethylamin), welches die Formel

C₆H₅-CH₂-CH(CH₃)-N(CH₃)-CH₂-C≡CH I

besitzt.

Zur Synthese von Deprenyl bzw. seiner Antipoden sind eine Reihe von Herstellungsverfahren bekannt, die sich sowohl mit der Herstellung von racemischem Produkt als auch den optischen Isomeren befassen. Als Ausgangsmaterial wird racemisches oder optisch aktives N, (x-Dimethylphenethylamin [C₆H₅-CH₂-CH(CH₃)-NH-CH₃ (= II)] verwendet.

In der CH-PS 393.306 wird u.a. die Herstellung von racemischem Deprenyl ausgehend von II unter Zuhilfenahme eines Metallierungsmittels wie Lithiumamid oder Phenylnatrium und nachfolgender zehnstündiger Alkylierung in siedendem Toluol mit 3-Brom-l-propin beschrieben. Derartige Reaktionen lassen sich im technischen Maßstab kaum durchführen.

Nach einem anderen chemischen Verfahren (NL-OS 6.605.956) läßt man II mit Propargylaldehyd reagieren und reduziert das entstandene Zwischenprodukt mit Aluminiumamalgam. Nachteile dieses Verfahrens liegen in der unter den Reaktionsbedingungen starken Polymerisationsneigung des Propargylaldehydes. Letzterer kann starke Hautirritationen verursachen. Des weiteren sind Quecksilber und dessen Salze starke Umweltgifte, die nur unter großem Aufwand entsorgt werden können.

Die in DE-AS 1.227.447 beschriebene Herstellung von Deprenyl durch Umsetzung von--Methylphenylethylchlorid mittels N-Methylpropinylamin im geschlossenen Rohr bei 80°C ist wegen der schlechten Ausbeute und der hohen Kosten des N-Methylpropinylamins im technischen Maßstab praktisch nicht zu realisieren. Die hohen Kosten der eingesetzten Reagenzien machen des Verfahren ökonomisch nicht sinnvoll.

Nach der DE-AS 1.227.447 wird weiter II mit 1,²-Dibrompropen bei 100°C 7 Stunden reagieren gelassen. Nicht umgesetztes Ausgangsmaterial wird durch Reaktion mit Benzoylchlorid in N,α-Dimethyl-N-benzoylphenethylamin überführt, welches vom gewünschten Endprodukt abgetrennt wird. Aus dem entstandenen N,α-Dimethyl-N-3-brom-2-propenylphenethylamin muß unter Einwirkung von alkoholisch-wäßriger Kalilauge Bromwasserstoff eliminiert werden, um die erwünschte Propinylverbindung zu erhalten. Dieses Verfahren ist mühsam, zeitaufwendig und für eine Produktion im technischen Maßstab nicht geeignet.

Schließlich wird in der DE-AS 1.227.447 (vgl. Beispiel 5) noch die Umsetzung von Propargylbromid mit der doppelten molaren Menge an N-(1-Phenylisopropyl)-methylamin in Abwesenheit eines Lösungsmittels bei 100°C beschrieben. Dieses Verfahren liefert zwar eine relativ gute Ausbeute, es eignet sich aber nicht für eine technische Synthese, da die Isolierung und Reinigung des Endproduktes sehr aufwendig ist.

Ähnliches gilt für die in Acta Pharm. Hung. 1992, 62, 201 (Seite 204) zitierte Umsetzung von N-(1-Phenylisopropyli∼methyl∼ amin mit Acetylen in Gegenwart von Paraformaldehyd und CuCl₂ (AT-PS 252.901). Bei dieser Reaktion muß das Selegilin durch Destillation gereinigt werden.

Auch für das nach H. Cabelled Compds. Radiopharm 1988, 25, 1 (Seite 7) hergestellte Selegilin ist ein aufwendiges Reinigungsverfahren erforderlich.

In der EP-PS 99.302 wird die Alkylierung von (R)-II mit 3-Brom-1-propin in einem zweiphasigen binären System von Benzol und wäßrigem Alkali bei einer Ausgangstemperatur von 60°C beschrieben. Das Arbeiten bei erhöhter Temperatur sowie die Verwendung von wäßriger Natronlauge verursachen jedoch durch die Anwesenheit von 3-Brom-1-propin Probleme, wie z.B. leichte und vermehrte Bildung von polymeren Produkten.

Gemäß einer weiteren Methode (EP-OS 344.675) wird in halogenhaltigen aliphatischen Kohlenwasserstoffen mit 3-Brom-1-propin unter Verwendung von Kaliumcarbonat alkyliert. Das Alkylierungsmittel und die Hilfsbase werden in einem Überschuß von mindestens 10 % verwendet. Die gesamte Menge 3-Brom-1-propin wird auf einmal oder in einem Zeitintervall von nur 5 Minuten zugegeben. Überschuß und Einmalzugabe sind für den Verlauf der Alkylierung nicht förderlich, denn die Ausbeuten an Selegilin-Hydrochlorid betragen nur knapp über 50 %. Außerdem ist die Verwendung von halogenhaltigen Kohlenwasserstoffen, wie insbesondere Chloroform, wegen der Kanzerogenität des Lösungsmittels aus Arbeits- sowie Umweltschutz-Gesichtspunkten nicht ungefährlich.

Diese bekannten Verfahren sind schwierig in den großtechnischen Maßstab zu übertragen und ermöglichen nicht die Herstellung von Selegilin-Hydrochlorid in guter Ausbeute.

Gegenstand der Erfindung ist ein Verfahren zur Herstellung von Selegilin-Hydrochlorid durch Umsetzen von R-(-)-N,α-Dimethylphenylethylamin mit 3-Brom-1-propin, welches darin besteht, daß man die Substanzen im Molverhältnis von etwa 2:1 bei 30 - 50°C in einem Lösungsmittelgemisch aus einem aromatischen Kohlenwasserstoff und Wasser in Abwesenheit eines Katalysators umsetzt und das Selegilin aus der organischen Phase isoliert und in das Hydrochlorid überführt.

Das Molverhältnis von II zu 3-Brom-1-propin soll so liegen, daß die Menge II gerade ausreicht, um den bei der Reaktion entstehenden Bromwasserstoff zu binden. In der Regel werden dazu etwas weniger als 2 Mole II pro Mol 3-Brom-1-propin benötigt. Ein gegebenenfalls nach Reaktionsende vorliegender Überschuß an II sollte neutralisiert werden, vorzugsweise mit HBr.

Die Reaktion wird bei 30 - 50°C, vorzugsweise bei 35 - 45°C durchgeführt. Die Reaktion ist in der Regel nach 5 Stunden beendet. Durch Kühlen kann man die Reaktionszeit verkürzen.

Die Reaktionstemperatur wird durch die Geschwindigkeit der Zugabe des 3-Brompropins geregelt.

Die Umsetzung erfolgt in einem Zweiphasensystem aus Wasser und einem aromatischen Kohlenwasserstoff wie Benzol, Toluol, Ethylbenzol, o-, m- oder p-Xylole oder trialkyliertes Benzol, wie Mesitylen. Toluol ist der am besten geeignete Kohlenwasserstoff.

Es ist wichtig, daß das im Verlauf der Alkylierung entstehende Bromid-Ion schnell durch die im Überschuß vorhandene organische Base gebunden und als leicht lösliches Salz möglichst rasch in die wäßrige Phase gelangt.

Die Menge an Wasser im Zweiphasensystem sollte über 10 % vorzugsweise 20 % oder mehr betragen. Mengen von über 30 % erbringen keine weiteren Vorteile.

Es ist ein besonderes Kennzeichen des Verfahrens, daß kein Katalysator benötigt wird.

Bei der Reaktion geht das entstehende Selegilin in die organische Phase. Nach Einengen der organischen Phase wird der Rückstand in einem polaren Lösungsmittel, wie Aceton, Methylethylketon oder anderen niedermolakularen Ketonen, aufgenommen. Die Lösung wird gegebenenfalls nach Zusatz von Aktivkohle filtriert. Anschließend wird mit Chlorwasserstoff das Hydrochlorid ausgefällt.

Aus der Mutterlauge läßt sich weiteres Selegilin-Hydrochlorid isolieren.

Aus der wäßrigen Phase läßt sich die Verbindung II nach Zugabe von Lauge extrahieren. Die Verbindung II kann ebenfalls für einen weiteren Reaktionsansatz verwendet werden.

Das neue Verfahren besitzt den Vorteil, daß es das Selegilin-Hydrochlorid in einer sehr guten Ausbeute und Reinheit liefert. Darüber hinaus ist das Verfahren einfach und ohne größeren Aufwand durchführbar. So werden beispielsweise keine höheren Temperaturen benötigt.

### Beispiel

Ein 500 ml Dreihalskolben, versehen mit Rückflußkühler, Rührer und Tropftrichter wurde mit Stickstoff gespült, 124 g (R)-(-)-N,α-Dimethylphenylethylamin eingefüllt und unter Rühren mit 160 ml Toluol und 40 ml Wasser auf 38 - 42°C erwärmt und unter intensivem Rühren innerhalb von 3 h 49,3 g reines 3-Brom-1-propin in der Weise zugetropft, daß die Temperatur 40 - 45°C nicht überschritt. Nach beendeter Zugabe wurde der Reaktionsansatz zur Vervollständigung der Umsetzung weitere 5 h bei 40 - 45°C gerührt und auf Raumtemperatur abkühlen gelassen, wobei sich 2 Phasen bildeten. Nach Trennung der beiden Phasen wurde in der organischen Phase der Gehalt an nicht umgesetztem (R)-(-)-N,α-Dimethylphenylethylamin bestimmt, die äquivalente Menge Bromwasserstoffsäure zugefügt, 15 min intensiv gerührt und danach 15 min stehen gelassen. Die Toluol-Phase wurde von der wäßrigen Phase getrennt und zweimal mit je 50 ml Wasser gewaschen. Die vereinigten wäßrigen Extrakte wurden gesammelt. Die Toluol-Phase wurde im Vakuum bei 50°C vollständig von Wasser und Toluol befreit. Der Rückstand wurde in 300 ml Aceton gelöst, unter Rühren mit 1 g Aktivkohle versetzt und im Vakuum filtriert. In die acetonische Lösung wurden unter Rühren und Kühlen 15,1 g Chlorwasserstoffgas bei < 30°C eingeleitet. Es stellte sich ein pH-Wert von ca. 2 ein. Der Reaktionsansatz wurde unter Rühren auf 20°C abgekühlt und 6 h lang weitergerührt. Die ausgefallenen Kristalle wurden unter Vakuum abgesaugt, mit 30 ml Aceton gewaschen und im Vakuum bei 70°C getrocknet. Man erhielt 81,7 g Selegilin-Hydrochlorid, Schmp. 142 - 145°C;
[α]_{D}²⁰ = 11,8°/c = 10, Wasser (Ausbeute 88 %)

Von der acetonischen Mutterlauge des Erstkristallisates wurden bei Normaldruck ca. 80 % des Lösungsmittels abdestilliert. Nach Abkühlen auf 20°C und mehrstündiger Kristallisation erhielt man ein zweites Kristallisat, das unter Vakuum abgesaugt mit wenig Aceton gewaschen und bei 70°C im Vakuum getrocknet wurde. Man erhielt weitere 8 g Selegilin-Hydrochlorid, das aus wäßrigem Aceton unter Zugabe von Aktivkohle umkristallisiert wurde. Es wurden 4,6 g Selegilin-Hydrochlorid erhalten, Schmp. 142 - 147°C; [α]_{D}²⁰ = 12°/c = 10, Wasser (Ausbeute 5 %).

Die vereinigten wäßrigen Lösungen wurden mit Natronlauge versetzt. Das (R)-(-)-N,α-Dimethylphenylethylamin wurde mit Toluol extrahiert. Die auf diese Weise gewonnene Base kann für den nächsten Ansatz zur Herstellung von Selegilin-Hydrochlorid wieder verwendet werden.

## Patentansprüche

1. Verfahren zur Herstellung von Selegilin-Hydrochlorid durch Umsetzen von R-(-)-N,α-Dimethyl-phenylethylamin mit 3-Brom-1-propin, dadurch gekenzeichnet, daß man die Substanzen im Molverhältnis von etwa 2:1 bei 30 - 50°C in einem Lösungsmittelgemisch aus einem aromatischen Kohlenwasserstoff und Wasser in Abwesenheit eines Katalysators umsetzt und das Selegilin aus der organischen Phase isoliert und in das Hydrochlorid überführt.

## Claims

1. A process for preparing selegiline hydrochloride by reaction of R-(-)-N,α-dimethylphenylethylamine with 3-bromo-1-propyne, which comprises reacting the substances in a molar ratio of approximately 2:1 at 30 - 50°C in a solvent mixture of an aromatic hydrocarbon and water in the absence of a catalyst and isolating the selegiline from the organic phase and converting it into the hydrochloride.

## Revendications

1. Procédé pour la préparation du chlorhydrate de sélégiline par réaction de la R-(-)-N,alpha-diméthyl-phényléthylamine avec le 3-bromo-1-propyne, caractérisé par le fait que l'on fait réagir ces substances, à un rapport molaire d'environ 2 : 1, à une température de 30 à 50°C, dans un mélange solvant consistant en un hydrocarbure aromatique et de l'eau, en l'absence de catalyseur, on isole la sélégiline de la phase organique et on la convertit en le chlorhydrate.
